# EUROPÄISCHE PATENTANMELDUNG

(11) **EP 2 103 751 A1**
(43) Veröffentlichungstag der Anmeldung: **23.09.2009**
(21) Anmeldenummer: 08153020.6
(22) Anmeldetag: 19.03.2008
(51) Int. Cl.: E04B 1/348, E04F 19/08, E04B 2/74, A47B 96/04, A47B 81/06, G06F 1/16, H04N 5/64, F16M 13/02, F16M 11/04, A61G 10/00, A61B 19/02

(54) **Einbaueinheit für Anzeigevorrichtungen**

(71) Anmelder: BrainLAB AG, 85622 Feldkirchen (DE)
(72) Erfinder: Steinle, Wolfgang, 80337, München (DE); Frielinghaus, Nils, 85551, Heimstetten (DE); Hamilton, Christoffer, 81371, München (DE)
(74) Vertreter: Schwabe - Sandmair - Marx

(57) **Zusammenfassung**

Die Erfindung betrifft eine Einbaueinheit für Anzeigevorrichtungen zum Einbau in einem Gebäude, mit zumindest einer Aufnahme (2) für eine medizintechnische Anzeigevorrichtung (3).

## Beschreibung

Die Erfindung betrifft eine Einbaueinheit für Anzeigevorrichtungen zum Einbau in einem Gebäude. Insbesondere betrifft sie eine Einbaueinheit für medizintechnische Anzeigevorrichtungen.

Aus modernen Operationssälen sind medizintechnische Anzeigevorrichtungen mit deren Hilfe Operationen geplant, vorbereitet, durchgeführt und besprochen werden können, kaum mehr wegzudenken. Normalerweise sind diese Anzeigevorrichtungen mit Rechnern verbunden, auf denen Patientendaten abgespeichert, bearbeitet und weitergeleitet werden können. Der Fachwelt sind prinzipiell zwei Möglichkeiten bekannt, wie Patientendaten angezeigt werden können. Einerseits können weitläufig bekannte Computereinheiten vorgesehen werden, jedoch haben diese einen großen Platzbedarf und nehmen daher Raum ein, der insbesondere in Operationssälen anderweitig Verwendung finden kann. Auch gestaltet sich eine Besprechung mit mehreren Personen vor einem herkömmlichen Computermonitor äußerst schwierig. Ferner sind herkömmliche Computersysteme sehr schmutzanfällig und daher ungeeignet für Operationssäle. Aus diesen Gründen geht man in letzter Zeit mehr und mehr auf nun verfügbare großformatige Flachbildschirme über, die beispielsweise an den Wänden der Operationssäle installiert werden können. Jedoch ist es nach wie vor nicht möglich, dass mehrere der Monitore, insbesondere wenn sie sich in unterschiedlichen Räumen befinden, ressourcensparend auf den gleichen Rechner zugreifen. Auch müssen für diese Flachbildschirme zunächst Orte ausgewählt werden, an denen sie an der Wand befestigt werden können, wobei entweder eine Aufhängevorrichtung an der Wand installiert werden muss oder, falls der Flachbildschirm in der Wand integriert werden soll, eine entsprechende Vertiefung in die Wand geschlagen werden muss. Dies bedeutet einen erheblichen Aufwand und Kosten und führt nicht zuletzt zur Verschmutzung des gesamten Operationssaals mit den entsprechenden Folgen.

Es ist die Aufgabe der Erfindung, eine Möglichkeit zur Installation einer medizintechnischen Anzeigevorrichtung in einem Gebäude, insbesondere in einem Operationssaal bereitzustellen, so dass eine Anzeigevorrichtung einfach, sauber und unkompliziert an dem für sie bestimmten Ort installiert werden kann.

Diese Aufgabe wird durch eine Einbaueinheit gemäß dem Anspruch 1 gelöst. Die Unteransprüche definieren dabei die bevorzugten Ausführungsformen der Erfindung

Die erfindungsgemäße Einbaueinheit umfasst zumindest eine Aufnahme für eine medizintechnische Anzeigevorrichtung, wobei die Einbaueinheit zum Einbau in einem Gebäude ausgestaltet ist. Mit anderen Worten wird schon beim Entwurf bzw. beim Bau des Gebäudes ein passender Ort ausgewählt, an dem später die Anzeigevorrichtung im Operationssaal angeordnet werden soll. So kann schon beim Bau des Gebäudes an dieser Stelle durch die Einbaueinheit eine Art Aussparung in dem Gebäude vorgesehen werden, so dass die Anzeigevorrichtung später platzsparend und sauber in die Aussparung eingelassen werden kann. Selbstverständlich kann die erfindungsgemäße Einbaueinheit auch für einen nachträglichen Einbau, sprich eine Nachrüstung in einem schon bestehenden Gebäude verwendet werden.

Dabei ist die Formulierung "zum Einbau in einem Gebäude" so zu verstehen, dass der Einbau bzw. Einbauort an beliebiger Stelle innerhalb des Gebäudes stattfinden bzw. sein kann. Das heißt, dass die Einbaueinheit in Zwischenwände, Außenwände, Decken, Fußböden oder ähnlichen Raumteilungselementen eingebaut werden kann. Darüber hinaus ist jedoch auch ein Einbau in sonstige Flächenelemente innerhalb des Gebäudes vorstellbar, wie etwa in Schrankwände oder -türen oder Zimmertüren.

Falls die spätere Anzeigevorrichtung und somit die Einbaueinheit in Gebäudeteilen selbst vorgesehen ist, wird um die plazierte Einbaueinheit herum dann das Gebäude, insbesondere Gebäude-Raumteilungs-Elemente in üblicher Weise errichtet. Dabei können die Gebäude-Raumteilungs-Elemente Gebäudewände, Gebäudedecken oder Gebäudefußböden sein.

Ferner ist es vorstellbar, dass die Anzeigevorrichtung in Elementen angeordnet werden soll, die nicht direkt zur Gebäudestruktur selbst gehören. Dies ist der Fall, wenn eine Gebäudetür, insbesondere eine Zimmertür oder ein Wandschrank, insbesondere die Wandschranktür mit einer Anzeigevorrichtung ausgestattet werden soll. In diese Elemente wird dann lediglich eine Aussparung vorgesehen, in die die Einbaueinheit dann einfach und unkompliziert eingesetzt wird, die später eine Anzeigevorrichtung aufnimmt. Eine direkte und platzraubende Montage der Anzeigevorrichtung an diesen Elementen ist dann nicht notwendig. Zusammenfassend lässt sich sagen, dass ein An- oder Einbau der erfindungsgemäßen Einbaueinheit überall dort möglich ist, wo im Gebäude Flächenelemente ausreichender Fläche vorhanden sind. Demzufolge ist hier beispielsweise auch an einen Einbau in oder an einen Schrank zu denken, auch wenn im Folgenden von Raumteilungselementen gesprochen wird.

Der dadurch entstandene Raum innerhalb der Einbaueinheit kann dann zur Aufnahme der medizintechnischen Anzeigevorrichtung genutzt werden. Vorteilhafterweise erstreckt sich die Ausnehmung über die gesamte Ausdehnung der Einbaueinheit, so dass die Einbaueinheit einem gewöhnlichen Tür- oder Fensterrahmen ähnelt.

Gemäß einer bevorzugten Ausführungsform der Erfindung erstreckt sich die Ausnehmung durch die gesamte Dicke des Gebäude-Raumteilungselements hindurch, so dass eine Art Fenster zwischen zwei Räumen entsteht. So können vorteilhafterweise an beiden Seiten der Einbaueinheit Anzeigevorrichtungen installiert werden, wobei diese insbesondere auf einfache Weise über Datenleitungen miteinander verbunden werden können. Dies ist unter anderem dann vorteilhaft, wenn beide Anzeigevorrichtungen zur Kommunikation von vor den Anzeigevorrichtung befindlichem Personal benutzt werden.

Ferner ist es denkbar, dass die Einbaueinheit an sich schon Zu- und/oder Abführungen für Signal- und Datenleitungen aufweist. Diese können an der Einbaueinheit schon so angeordnet sein, dass die entsprechenden Leitungen bzw. Kabel in dem Gebäude-Raumteilungselement verlaufen, also schon bei der Planung und Errichtung des Gebäudes in dem Gebäude-Raumteilungselement vorgesehen werden können. So ist es möglich, dass die Einbaueinheit schon Anschlüsse in der Art von Steckdosen bereitstellt, an denen eine bzw. mehrere Anzeigevorrichtungen einfach angesteckt werden. Somit wird die Installation der Anzeigevorrichtungen zusätzlich vereinfacht, wobei die gesamte Installation nach dem Einbau einen ästhetischen und aufgeräumten Eindruck hinterlässt. Diese Signalleitungen können dann beispielsweise zu einem zentral angeordneten Computer führen oder zu anderen Stockwerken oder entfernt gelegenen Räumlichkeiten, in denen ebenfalls Anzeigevorrichtungen vorhanden sind. Dieselbe Lösung bietet sich ebenfalls für Stromkabel für die Energieversorgung der Anzeigevorrichtungen an.

Da die Elektronik der Anzeigevorrichtungen üblicherweise große Mengen an Wärmeleistung abgeben, können in einer weiteren bevorzugten Ausführungsform in oder an der Einbaueinheit Zu- und/oder Abführungen für Frisch- bzw. Abluft vorgesehen sein. So kann einerseits durch Luftschlitze zum Operationssaal hin eine Luftzirkulation zwischen dem entsprechenden Operationssaal und der Anzeigevorrichtung vorgesehen sein, jedoch stellt insbesondere die Luftführung in Kanälen innerhalb des Gebäude-Raumteilungselements eine elegante Lösung dar, da so der entsprechende Operationssaal nicht mit einer Luftströmung mit entsprechender Verschmutzungsgefahr beaufschlagt wird. So werden die Anzeigevorrichtungen vorteilhafterweise schon innerhalb des Gebäude-Raumteilungselements "hinterlüftet".

Bei manchen Anwendungen ist eine besonders einfache Ausgestaltung der Einbaueinheit mit den entsprechenden Anzeigevorrichtungen wünschenswert. So kann dezentralisiert in oder an jeder Einbaueinheit eine Rechnereinheit vorgesehen sein, die eine oder mehrere Anzeigevorrichtungen der Einbaueinheit mit Daten speist und auch zur Kommunikation zwischen den beiden Anzeigeeinheiten dienen kann. Da Gebäude-Raumteilungselement üblicherweise mehr als die doppelte Tiefe von gewöhnlichen Flachbildschirmen aufweisen, können an den gegenüberliegenden Seiten der Einbaueinheit, sprich an beiden Flächen des Gebäude-Raumteilungselements voneinander abgewandte Anzeigevorrichtungen vorgesehen sein. Selbst bei Installationen von zwei Anzeigevorrichtungen in einer Einbaueinheit ist zwischen den Anzeigevorrichtungen noch genügend Raum vorhanden, um darin eine Rechnereinheit anzuordnen.

Besonders vorteilhaft, insbesondere in Verbindung mit der Kommunikation von Personal über die Anzeigevorrichtungen im Gebäude ist das Ausstatten der Einbaueinheit mit ein oder mehreren Kamera(s), die den Raum vor den Anzeigevorrichtungen aufnehmen. So kann das Personal in der Art von Videotelefonie miteinander kommunizieren und sogleich Daten bzw. Bilder auf einfache oder ökonomische Weise austauschen und diese besprechen. Auch können diese oder weitere Kameras so ausgerichtet werden, dass sie die Oberfläche der Anzeigevorrichtungen aufnehmen, um etwaige Verschmutzungen auf der Bildschirmioberfläche auszumachen. Wenn beispielsweise auf der Anzeigevorrichtung ein Testbild gezeigt wird, kann dies von der Kamera aufgenommen werden, wobei Verschmutzungen der Anzeigeoberfläche durch Differenzen zwischen angezeigtem und dem aufgenommenem Bild ausgemacht werden. In einen weiteren Schritt kann dann ein solcher verschmutzter Bereich durch die Anzeigevorrichtung selbst markiert werden, so dass beispielsweise Reinigungspersonal über den Verschmutzungszustand der Anzeigevorrichtung informiert wird.

Auch kann an der Einbaueinheit eine Projcktionseinheit vorgesehen sein, die beispielsweise eine virtuelle Tastatur oder ein Bedienfeld auf eine Fläche nahe der Anzeigevorrichtung projiziert, wobei insbesondere in Verbindung mit einer die Position des Personals aufnehmenden Kamera eine virtuelle berührungslose Bedienung der Anzeigevorrichtung möglich wird. Dies ist insbesondere bei sterilen Bedingungen in Operationssälen von Vorteil. Jedoch kann die Anzeigevorrichtung selbst schon in bekannter Weise als "Touchscreen" ausgebildet sein, so dass das Personal die Bedienung über die Anzeigenoberftäche vornimmt.

Um die Anzeigevorrichtung bei Nichtgebrauch zu schützen, kann in oder an der Einbaueinheit eine Art Schutzrollo vorgesehen sein, welches im Ruhemodus der Anzeigevorrichtung über diese gezogen wird. Dabei kann das Schutzrollo zusätzlich Reinigungsmittel umfassen, welche bei der Bewegung des Schutzrollos über die Anzeigenoberfläche diese reinigen.

Die Einbaueinheit kann von beliebiger Größe ausgestaltet sein, so dass eine darin angeordnete Anzeigevorrichtung den zur Verfügung stehenden Raum sowohl in der Tiefe und/oder in der flächenmäßigen Erstreckung nicht ganz ausfüllt. Um ein möglichst ansprechendes optisches Äußeres zu erzielen, kann die Anzeigeeinheit so in der Einbaueinheit ausgerichtet sein, dass sie plan mit der entsprechenden Oberfläche des Gebäude-Raumteilungselements abschließt.

Die Anzeigevorrichtung kann dabei jeder beliebige und weitläufige bekannte Flachbildschirm sein, jedoch ist bei den oben genannten Anwendungsfällen die Verwendung eines digitalen medizintecgnischen Lichtkastens bzw. einer digitalen Lightbox von Vorteil, da sie genau für diese Anwendungsfälle konzipiert wurde.

Ferner ist es denkbar, dass die Anzeigevorrichtungen verschwenk- und/oder verdrehbar an der Einbaueinheit angeschlagen sind. Auf diese Weise ist es möglich, Spiegelungen an der Anzeigenoberfläche durch schräge Draufsicht auf diese zu vermeiden, indem die Anzeigevorrichtung immer so ausgerichtet wird, dass das Personal eine senkrecht auf die Anzeigeoberfläche stehende Blickrichtung hat. Man kann sich aber ebenso vorstellen, dass in derselben Einbaueinheit sowohl ein bekannter analoger Lichtkasten als auch eine digitale Lightbox so angeortnet sind, dass deren Anzeigeflächen voneinander abgewandt sind und dieser Zusammenschluss über eine Drehvorrichtung so verschwenkbar ist, dass das Personal zwischen der digitalen Lightbox und dem analogen Lichtkasten durch Verschwenken des gesamten Zusammenschlusses wählen kann. Eine andere Möglichkeit zum Schutz der Anzeigevorrichtung stellt neben dem Vorsehen eines Rollos das Verschwenken der Einzeigevorrichtung dar. Diese kann nämlich bei Nichtgebrauch so verschwenkt werden, dass die Anzeigenoberfläche vom Operationssaal abgewandt im Inneren der Einbaueinheit liegt.

Wie schon oben angedeutet, kann in der erfindungsgemäßen Einbaueinheit lediglich eine Anzeigevorrichtung vorgesehen sein, jedoch ist bei optimaler Anordnung der Einbaueinheit im Gebäude, also beispielsweise zwischen zwei Operationssälen eine Anbringung von zwei einander abgewandten Anzeigeeinheiten zu je einem Operationssaal hin denkbar. Dadurch können neben der Verwendung einer einzigen Einbaueinheit für zwei Anzeigevorrichtungen Ressourcen durch die gemeinsame Verwendung einer einzigen Rechnereinheit und deren Energieversorgung auf zwei Anzeigeeinheiten verteilt werden.

Zudem kann an der Einbaueinheit ein Bedienfeld in Form einer Tastatur angeordnet sein, welche Eingaben des Personals ermöglicht.

Falls hinter der einen bzw. zwischen den zwei Anzeigeeinheiten in der Einbaueinheit noch Raum vorhanden ist, so kann dieser etwa in Form eines Regals oder Wandschranks genutzt werden. So können mehrere Ablagemöglichkeiten bzw. Fächer mit Stauraum in der Einbaueinheit vorgesehen sein. Die Anzeigeeinheit kann dann ähnlich wie eine Schranktür aufgeschwenkt werden, um dem Personal Zugriff zum Stauraum in der Einbaueinheit zu ermöglichen. Die Anzeigeeinheit kann dann in der Art einer Tresortüre ein Zahlenschloss darstellen und nur berechtigtem Personal Zugriff gewähren.

Es ist ferner denkbar, dass an der Einbaueinheit Anschlüsse vorgesehen sind, die zum Operationssaal hin ausgerichtet sind, so dass ein Anschluss externer Geräte über die Einbaueinheit an die in der Einbaueinheit befindliche Anzeigeeinheit oder ein daran angeschlossenen Rechnersystem möglich ist. Dies kann beispielsweise ein Videoausgang oder -eingang sein. Im Extremfall können dabei nur Anschlüsse von der Einbaueinheit bereitgestellt werden, während sie keine Anzeigevorrichtungen umfasst.

Ferner kann die Einbaueinheit als starrer Rahmen mit festen Ausmaßen ausgestaltet ist, jedoch ist es auch denkbar, dass die Einbaueinheit in ihren Maßen variabel ist. Beispielsweise können durch eine Art Schienensystem einzelne Segmente der Einbaueinheit zueinander verschiebbar sein, um so beispielsweise universell an verschiedene Dicken verschiedener Gebäude-Raumteilungselemente anpassbar oder für verschiedene Formate von Anzeigeeinheiten einsetzbar zu sein. Auch wäre ein Teleskopieren einzelner Segmente zueinander denkbar. Insbesondere ist dabei die Teilung der Einbaueinheit in acht Segmente vorstellbar, wobei jeweils vier Segmente auf jeder Wandseite angeordnet sind und diese jeweils zueinander an den Seitenhalbierenden der vier Rahmenseiten der Einbaueinheit einander zugeordnet sind.

Auch ist es möglich, die erfindungsgemäße Einbaueinheit mit einem Scanner oder/und einem Drucker auszustatten um dem Personal direkte Eingabe bzw. Ausgabe von Daten zu ermöglichen. So kann etwa im Randbereich der Anzeigevorrichtung ein Scanner vorgesehen sein, der beispielsweise vom Personal eingelegte Papierdokumente oder Röntgenbilder einzieht, welche daraufhin durch die Anzeigevorrichtung dargestellt werden. Im Gegenzug ist auch ein Drucker vorstellbar, beispielsweise ebenfalls am Rand der Anzeigevorrichtung angeordnet, welcher die vom Personal gewünschten Dokumente, die ebenfalls durch die Anzeigevorrichtung dargestellt werden können, ausdruckt.

Die Erfindung beschränkt sich nicht nur auf die Verwendung als Einbaueinheit für medizintechnische Anzeigevorrichtungen, sie kann vielmehr auch in anderen Bereichen eingesetzt werden, beispielsweise beim Neubau eines Wohnhauses oder Hotels, wobei darin ein Fernseher oder Computermonitor angeordnet werden soll. Ebenso ist, wie schon oben dargesellt, der Einbau in eine Gebäudedecke, den Fußboden oder gar in eine Zimmertür oder Schranktür vorstellbar.

Die vorliegende Erfindung wird in einer Ausführungsform anhand der beiliegenden Figuren näher beschrieben, sie kann dabei einzelne Merkmale sowie jedwede sinnvolle Merkmalskombination umfassen. Gezeigt wird in
- Figur 1:: eine erfindungsgemäße Einbaueinheit im eingebauten Zustand in einer Wand
- Figur 2:: die in Figur 1 gezeigte Einbaueinheit in Kombination mit einer Kamera, einem Projektor und einer Rechnereinheit
- Figur 3:: die in der Figur 1 gezeigte Einbaueinheit mit eingesetzter Anzeigevorrichtung.

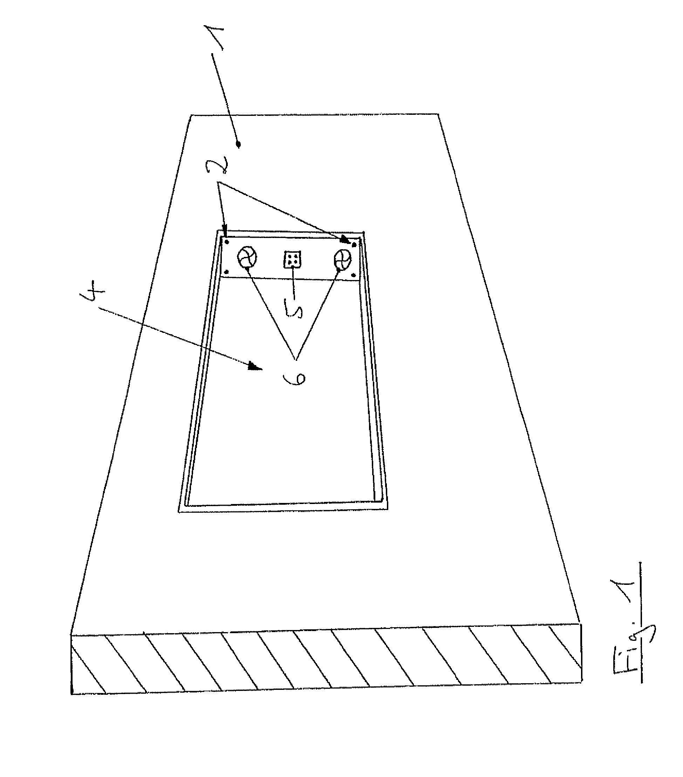

In der Figur 1 ist eine Einbaueinheit zu sehen, die bereits in eine Gebäudewand 1 eingesetzt bzw. eingebaut wurde, wobei dadurch eine Art rechteckiger Durchbruch bzw. Ausnehmung 4 in der Gebäudewand 1 entsteht. Die Einbaueinheit umfasst dabei mehrere Aufnahmen 2, an denen später Anzeigevorrichtungen 3 an der Einbaueinheit befestigt werden können. Im vorliegenden Fall, da hier die Einbaueinheit zu beiden Seiten hin offen ist, können von beiden an die Wand 1 angrenzenden Räumen Anzeigenvorrichtungen 3 in die Einbaueinheit eingesetzt werden. Ferner sind in der Einbaueinheit Zu- und/oder Abführeinrichtungen 5 für Signalleitungen und Stromzufuhr ebenso wie Zu- und/oder Abführeinrichtungen 6 für Frisch- bzw. Abluft vorgesehen. Somit wird die Anzeigeeinheit 3 nach dem Einsetzen in die Einbaueinheit mit diesen Anschlüsse verbunden, wonach keine störenden und schmutzanfälligen Kabel bzw. Kanäle zur Anzeigevorrichtung 3 an der Wand 1 entlang geführt werden müssen. Die gesamte Installation gibt so ein sowohl ästhetisch ansprechendes als auch ein schmutzunanfälliges und pflegeleichtes Bild ab.

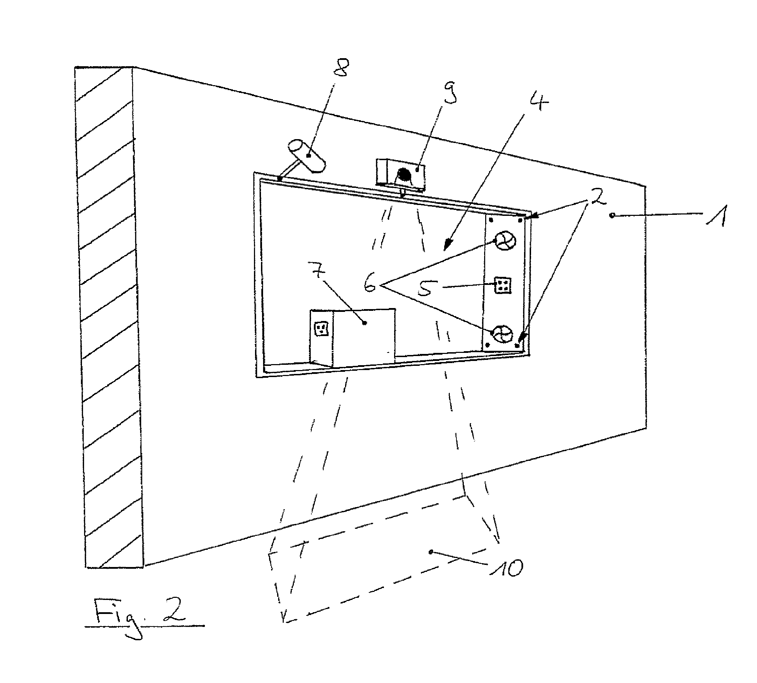

Bei der in der Figur 2 gezeigten Ausführungsform wurde die schon in der Figur 1 gezeigte Einbaueinheit um weitere Merkmale erweitert. So umfasst sie nunmehr ferner eine Recheneinheit 7, die mit den Anzeigevorrichtungen 3 und auch mit der Steckdose 5 der Einbaueinheit verbunden werden kann, um sowohl eine Datenkommunikation zwischen den zwei Anzeigeeinlleiten 3 als auch zu anderen Orten hin zu ermöglichen. Es können die Anzeigeeinheiten 3 jedoch auch direkt mit der Steckdose 5 verbunden werden.

Ferner umfasst die Einbaueinheit eine Projektionseinheit 9, die vor der Einbaueinheit bzw. der Anzeigevorrichtung 3 eine virtuelle Tastatur 10 auf den Fußboden projiziert. Zusammen mit der an der Einbaueinheit angeordneten Kamera 8 wird eine virtuelle Bedienung durch das Personal ermöglicht. Die Kamera 8 nimmt nämlich das Umfeld vor der Einbaueinheit bzw. der Anzeigevorrichtung 3 auf, also auch die Position und die Bewegungen des vor der Anzeigevorrichtung 3 befindlichen Personals. So kann beispielsweise ein Chirurg steril arbeiten, da er lediglich mit den Füßen die Bedienung der Anzeigevorrichtung 3 vornimmt und nicht die Oberfläche der Anzeigevorrichtung 3 mit den Händen berühren muss.

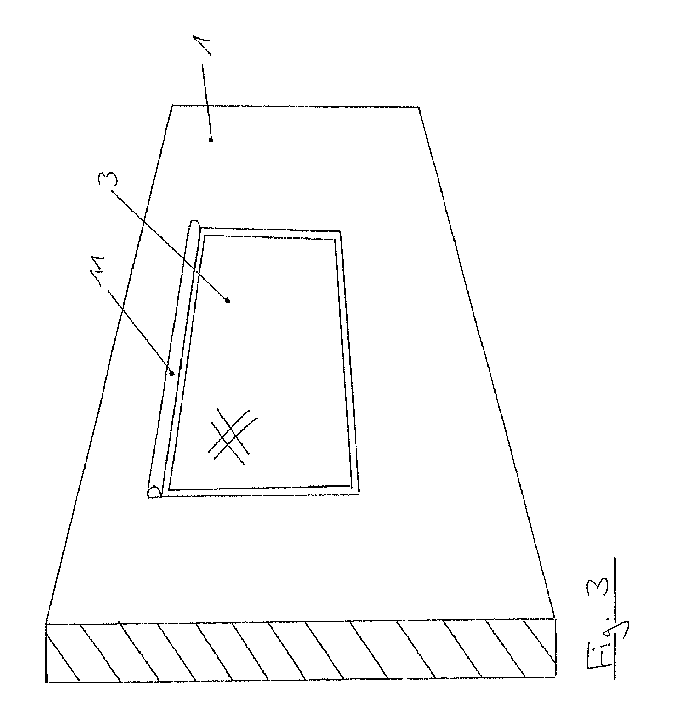

In der Figur 3 ist eine in die Einbaueinheit eingesetzte Anzeigevorrichtung 3 zu sehen, die bündig mit der Wandoberfläche der Wand 1 abschließt Alle Anschlüsse bzw. Zu- und/oder Abführungen von Datenleitungen oder Kühlluftkanälen befinden sich auf der Rückseite der Anzeigevorrichtung 3 und verlaufen innerhalb der Wand 1. Zum Schutz der Anzeigenoberfläche ist eine Schutzeinrichtung 11 in Form eines Rollos oberhalb der Anzeigevorrichtung 3 angeordnet. Bei Nichtbenutzung oder in einem Ruhemodus kann dieses Rollo 11 vor der Anzeigevorrichtung 3 heruntergezogen werden und schützt somit die Anzeigenoberfläche. Gleichzeitig reinigen beim Herunterziehen und beim Aufziehen der Schutzeinrichtung 11 Reinigungsmittel auf der Innenseite der Schutzeinrichtung 11 die Anzeigenoberfläche.

## Patentansprüche

1. Einbaueinheit für Anzeigevorrichtungen zum Einbau in einem Gebäude, mit zumindest einer Aufnahme (2) für eine medizintechnische Anzeigevorrichtung (3).

2. Einbaueinheit nach dem Anspruch I, **dadurch gekennzeichnet, dass** die Anzeigevorrichtung zum Einbau in ein Gebäude-Raumteilungselement ausgestaltet ist.

3. Einbaueinheit nach dem Anspruch 2, **dadurch gekennzeichnet, dass** die Anzeigevorrichtung zum Einbau in eine Gebäudewand (1) ausgestaltet ist.

4. Einbaueinheit nach dem Anspruch 2, **dadurch gekennzeichnet, dass** die Anzeigevorrichtung zum Einbau in eine Gebäudedecke und/oder einen Gebäudefußboden und/oder eine Zimmertür ausgestaltet ist.

5. Einbaueinheit nach dem Anspruch 1, **dadurch gekennzeichnet, dass** die Anzeigevorrichtung zum Einbau in einen Schrank, insbesondere eine Schranktür ausgestaltet ist.

6. Einbaueinheit nach einem der Ansprüche 3 bis 5, **dadurch gekennzeichnet, dass** innerhalb der Einbaueinheit eine hinter der Oberfläche des Gebäude-Raumteilungselements angeordnete Ausnehmung (4) vorgesehen ist.

7. Einbaueinheit nach einem der Ansprüche 3 bis 6, **dadurch gekennzeichnet, dass** sich die Ausnehmung (4) über die gesamte Ausdehnung der Einbaueinheit in der Oberflächenebene des Gebäude-Raumteilungselements erstreckt.

8. Einbaueinheit nach einem der Ansprüche 6 oder 7, **dadurch gekennzeichnet, dass** sich die Ausnehmung (4) durch die gesamte Dicke des Gebäude-Raumteilungselements hindurch erstreckt.

9. Einbaueinheit nach einem der Ansprüche 2 bis 8, **dadurch gekennzeichnet, dass** die Einbaueinheit zumindest eine insbesondere zwischen den Oberflächen des Gebäude-Raumteilungselements angeordnete Zu- und/oder Abführungseinrichtung (5) für Energieversorgungs- und/oder Signalleitungen umfasst.

10. Einbaueinheit nach einem der Ansprüche 1 bis 9, **dadurch gekennzeichnet, dass** die Einbaueinheit zumindest eine insbesondere zwischen den Oberflächen des Gebäude-Raumteilungselements angeordnete Zu- und/oder Abführungseinrichtung (6) von Frisch- bzw. Abluft umfasst.

11. Einbaueinheit nach einem der Ansprüche 1 bis 10, **dadurch gekennzeichnet, dass** die Einbaueinheit eine Rechnereinheit (7), insbesondere zur Verarbeitung der durch die Anzeigevorrichtung (3) darzustellenden Daten umfasst.

12. Einbaueinheit nach einem der Ansprüche 1 bis 11, **dadurch gekennzeichnet, dass** die Einbaueinheit eine Kamera (8) umfasst, die zumindest einen Teil des Raumes vor der Anzeigevorrichtung (3) und/oder die Anzeigevorrichtung (3) selbst erfasst.

13. Einbaueinheit nach einem der Ansprüche 1 bis 12, **dadurch gekennzeichnet, dass** die Einbaueinheit eine Projektionseinheit (9) umfasst, welche ein Bedienungsfeld, insbesondere eine virtuelle Tastatur (10) in der Umgebung der Anzeigevorrichtung (3), insbesondere auf den im Bereich der Anzeigevorrichtung befindlichen Fußboden projiziert.

14. Einbaueinheit nach einem der Ansprüche 1 bis 13, **dadurch gekennzeichet, dass** die Einbaueinheit eine Schutzeinrichtung (11) für die Anzeigevorrichtung, insbesondere ein Schutzrollo umfasst, das bei Bedarf vor die Anzeigevorrichtung gezogen werden kann und im Speziellen dabei die Anzeigefläche der Anzeigevorrichtung (3) reinigt.

15. Einbaueinheit nach einem der Ansprüche 1 bis 14, **dadurch gekennzeichnet, dass** sich die Einbaueinheit im Gebäude-Raumteilungselement im Wesentlichen quaderförmig erstreckt, insbesondere in der Ebene des Gebäude-Raumteilungselements im Wesentlichen die gleiche Erstreckung aufweist wie die Anzeigevorrichtung (3).

16. Einbaueinheit nach einem der Ansprüche 1 bis 15, **dadurch gekennzeichnet, dass** die Einbaueinheit, insbesondere eine daran angeordnete Anzeigevorrichtung (3) plan mit zumindest einer Oberfläche des Gebäude-Raumteilungselements abschließt.

17. Einbaueinheit nach einem der Ansprüche 1 bis 16, **dadurch gekennzeichnet, dass** die Anzeigevorrichtung (3) ein digitaler, medizintechnischer Lichtkasten bzw. eine digitale Lightbox ist.

18. Einbaueinheit nach einem der Ansprüche 1 bis 17, **dadurch gekennzeichnet, dass** die Anzeigevorrichtung (3) verschwenk- und/oder verdrehbar an der Einbaueinheit angeschlagen ist.

19. Einbaueinheit nach Anspruch 18, **dadurch gekennzeichnet, dass** die Einbaueinheit einen digitalen Lichtkasten und einen, insbesondere den digitalen Lichtkasten abgewandten, analogen Lichtkasten umfasst, die im Speziellen verschwenk-und/oder verdrehbar dem Benutzer zugewandt werden können.

20. Einbaueinheit nach einem der Ansprüche 18 oder 19, **dadurch gekennzeichnet, dass** die Anzeigevorrichtung (3) in eine vom Benutzer abgewandte Ruhestellung verschwenkt und/oder verdreht werden kann.

21. Einbaueinheit nach einem der Anspruche 1 bis 20, **dadurch gekennzeichnet, dass** die Einbaueinheit mindestens eine, vorzugsweise zwei Anzeigevorrichtungen (3) umfasst.

22. Einbaueinheit nach Anspruch 21, **dadurch gekennzeichnet, dass** die Anzeigevorrichtungen (3) jeweils an den gegenüberliegenden Oberflächen des Gebäude-Raumteilungselements angeordnet sind.

23. Einbaueinheit nach einem der Ansprüche 1 bis 22, **dadurch gekennzeichnet, dass** die Einbaueinheit und/oder die Anzeigevorrichtungen (3) ein Bedienfeld, insbesondere eine Tastatur umfasst.

24. Einbaueinheit nach einem der Ansprüche 1 bis 23, **dadurch gekennzeichnet, dass** die Anzeigevorrichtung (3) mit einer Rechnereinheit (7) signaltechnisch verbunden ist, vorzugsweise über die Rechnereinheit (7) signaltechnisch mit anderen Anzeigevorrichtungen verbunden ist.

25. Einbaueinheit nach einem der Ansprüche 1 bis 24, **dadurch gekennzeichnet, dass** die Anzeigevorrichtung (3) und/oder die Rechnereinheit (7) mit externen Systemen über eine Signalleitung, insbesondere eine im Gebäude-Raumteilungselement verlegte Signalleitung verbunden ist.

26. Einbaueinheit nach einem der Ansprüche 1 bis 25, **dadurch gekennzeichnet, dass** der nicht von der Anzeigevorrichtung (3) ausgefüllte Raum der Einbaueinheit als Lagerungs- bzw. Ablagebehältnis ausgebildet ist.

27. Einbaueinheit nach einem der Ansprüche 1 bis 26, **dadurch gekennzeichnet, dass** die Einbaueinheit einen Videoausgang umfasst.

28. Einbaueinheit nach einem der Ansprüche 1 bis 27, **dadurch gekennzeichnet, dass** die Einbaueinheit ein variable Breite aufweist, um an unterschiedliche Einbaudicken angepasst zu werden.
